# EUROPEAN PATENT APPLICATION

(11) **EP 4 116 203 A1**
(43) Date of publication of application: **11.01.2023**
(21) Application number: 21382610.0
(22) Date of filing: 06.07.2021
(51) Int. Cl.: B65B 25/00, B65B 31/02

(54) **METHOD, CONTAINER AND INSTALLATION FOR THE TREATMENT, STORAGE AND INTEGRAL WASTE MANAGEMENT**

(71) Applicant: Martinez Martinez, José, 29602 Marbella (ES)
(72) Inventor: Martinez Martinez, José, 29602 Marbella (ES)
(74) Representative: Hoffmann Eitle

(57) **Abstract**

The present invention relates to a method and a facility for integral waste treatment, storage, and management, which comprises: crushing and compacting previously sterilized waste so as to form a compact block of waste, enclosing the compact block of waste inside a flexible bag, applying a vacuum inside the bag, introducing the compact block of waste into a rigid container, hermetically closing the container, and applying an inertization substance on the flexible bag inside the hermetically closed rigid container, such that the definitive storage of the waste inside the container is maintained under vacuum and in the absence of biological or chemical activity. The invention also relates to a waste storage container, enclosing a biologically and chemically inertized compact block of waste, such that the definitive storage of the waste inside the container is maintained in the absence of biological or chemical activity.

## Description

### Technical Field and Object of the Invention

The present invention relates to a technological process for providing a definitive solution to the serious environmental problem affecting the health of human beings, land flora and fauna, as well as rivers, seas, oceans, and the atmosphere of the planet.

Specifically, the invention relates to a method, a container, and a facility for integral waste treatment, storage, and management.

The technology developed in this invention provides a radical and definitive solution to the problems derived from waste treatment and storage in landfills, providing a technique which obtains a synergistic effect resulting from the combination of a physical, chemical, and biological process, achieving waste treatment and storage without any type of emission into the environment such as: gases, liquids, odors, or hazardous particles, and without causing waste accumulations in landfills which may pollute the environment.

### State of the Art

Human activity involves the generation of significant amounts of waste. The abandonment or improper management of waste produces considerable impacts on the environment and causes water, land, and air pollution, as well as contributes to climate change, affecting ecosystems and human health. Therefore, the controlled treatment of this waste is compulsory and essential within an economy and industry that are sustainable with life and the environment.

In Spain, about 40 million tons of municipal waste are generated daily. In the European Union, fifty percent (50%) of waste are organic waste, and in the specific case of Spain, each Spanish person produces about 600 kg of garbage a year and forty-four percent (44%) of household waste are organic waste. In Spain, 53.7% of total municipal waste reach a landfill. The cost of the waste collection, elimination, and treatment service in Spain in 2017 exceeded four billion Euros (4630 million Euros) and revenues from population dues was slightly more than three billion Euros (3457 million Euros), with waste management therefore generating a deficit of more than one billion Euros (1173 million Euros).

In addition to the economical aspect, poor garbage management causes soil and landscape degradation, contaminates aquifers, rivers, lakes, and oceans, and has a direct and negative effect on health and biodiversity. About sixty percent (60%) of the volume and thirty percent (30%) of the weight of garbage bags are disposable receptacles. Many studies indicate that one-third of all plastic waste ends up in the soil or fresh water.

Plastic is an extremely long-lasting material which is dispersed very easily, so its amount in the ocean increases continuously.

On average, 8 million tons of plastic are dumped into the oceans each year, which is the equivalent to emptying a plastic-filled garbage truck every minute. If this trend is not changed, the earth's oceans will have 1 ton of plastic for every 3 tons of fish by 2025, and by 2050 there will be more plastics than fish.

There is a huge demand for plastic raw material and the global plastic production has increased from 2 million tons in 1950 to about 400 million tons in 2018. It is difficult to get an idea of this dimension which is equivalent to 13 million 30-ton trailer-tank trucks. The prediction is that demand will continue to grow until exceeding 1000 million tons by 2050.

This waste is driven by marine currents and has formed plastic islands or concentrations of considerable dimensions, with the largest one being found in the North Pacific (between the Californian coast and Hawaii) and is known as the "Great Pacific Garbage Patch" (GPGP). It measures more than a million square kilometers, exceeding the surface area of Spain, France, and Germany combined. Although the GPGP is the largest garbage island, it is not the only one, since one more can be found in the Pacific Ocean, two in the Atlantic Ocean, and another in the Indian Ocean. The Mediterranean sea is considered the 6^{th} greatest accumulation region for plastic waste. It holds only 1% of the world's waters but concentrates 7% of all microplastics (particles smaller than 5 millimeters in diameter) of the planet.

One-third of all plastic waste ends up in the soil or bodies of fresh water. It is estimated that land pollution by microplastics is 4 to 23 times higher than marine pollution. One example of this is the pollution caused by textile fibers which are retained in the slurries of waste waters that are furthermore often used as fertilizers, which translates into tons of microplastics deposited in soils.

It is calculated that up to 30 million (30,000,000) plastic bottles and cans are dumped every year in Spain alone. Furthermore, it is estimated that the degradation time of plastics in the ocean is much longer than on land since they are less exposed to sunlight, high temperatures, and oxygen.

Some studies estimate that the amount of plastic reaching the oceans directly or through rivers every year is between 5.95 and 15.11 million tons (5,950,000,000 - 15,110,000,000 kg), an enormous volume of garbage that the currents then move across virtually the entire planet.

It is calculated that there are more than two-hundred thousand tons (236,000 tons) of plastic particles floating on the surface of the different seas of the world. Most of this plastic disintegrates into particles of less than five millimeters (<5 mm), known as microplastics, and these microplastics break down further until they become nanoparticles (less than 0.1 micrometers in size).

However, in addition to being present in oceans and on land, microplastics enter living beings through the air that is breathed in and through the foods and drinks consumed. A recent study, "No Plastic in Nature: Assessing Plastic Ingestion from Nature To People" (prepared by Dalberg Advisors), based on a study requested by the WWF and performed by the University of Newcastle, suggests that people are consuming about 2000 tiny pieces of plastic every week, about 21 grams a month, and just over 250 grams a year. This is the equivalent to the weight of a credit card a week. The study indicates that the specific effects of microplastic ingestion on human health still remain unclear.

Solid waste most commonly end up in landfills or sanitary landfills. Controlled landfills can be defined as a site for eliminating waste by means of surface or underground deposition, in which waste is accumulated by means of controlled operations and with a suitable design, where the waste is pretreated, sorted, and compacted. However, landfills also entail a risk for the environment.

The particular case of landfills for electronic waste, particularly in Africa and Asia, constitutes a problem of catastrophic dimensions for the environment and human health.

Environmental pollution through poor waste management and control in landfills occurs as the result of the uncontrolled accumulation of garbage and the appearance of leachates. Leachates contain high concentrations of organic and inorganic contaminants, including humic acids, organic substances, nutrients, and heavy metals (such as lead or mercury), as well as electrical conductivity-increasing inorganic salts, and infectious agents. These leachates severely pollute the ecosystem (both land and water).

Furthermore, atmospheric pollution also occurs in landfills due to the conversion of the biodegradable waste fraction into a biogas that generates odors, noises, fire, and even explosions. This biogas produced as a result of the decomposition and fermentation of organic matter is a mixture of methane gas (CH₄) and carbon dioxide (CO₂) and is responsible for global warming. Methane is a greenhouse gas having a global warming potential between 21 and 25 times greater than CO₂.

Lastly, landfills also present a health risk as a result of the accumulation of vectors (rodents, birds, insects, etc.) which transmit diseases (virus, bacteria, etc.). Moreover, waste incineration, which is another form of waste treatment, is neither a renewable nor a clean technology, since household garbage is not a renewable resource, and the combustion thereof generates ashes and emissions of greenhouse gases, substances hazardous to health and the environment, in addition to increasing the pollutant emission rate of countries, therefore increasing the monetary amounts countries have to pay based on their emissions.

Therefore, waste does not disappear with combustion; it is only transformed into gases, liquids, and ashes, in addition to increasing the overall expenditure of the process. The emission of hazardous hydrocarbon particles and ashes, such as sulfur oxide, greenhouse gases, mercury, zinc, cadmium, nickel, chromium, arsenic, and nitrogen, among others, pollute water, land, and air. One of the most important concerns is the generation and release of dioxins and furans which are highly toxic and can travel long distances.

Furthermore, incineration is an expensive treatment since the sale of energy obtained with incineration at market prices is not enough to fund its operation.

Therefore, as a waste management system, incineration is also the most expensive technology, being up to between thirty-five percent and fifty percent (between 35% and 50%) more expensive than a landfill (even the most modern ones), depending on the region in which it is installed.

The following table shows the serious environmental impact of incineration and landfill storage in comparison with the present invention:

| **POLLUTION** | | | **MONETARY CONSIDERATIONS** |
|---|---|---|---|
| **INCINERATION AND LANDFILL:** | | **INVENTION:** | |
| Toxic gases | Tons x 10⁶ | | |
| Greenhouse gases | Tons x 10⁶ | NONE | |
| Furans and leachates | Tons x 10⁶ | NONE | |
| Hazards for human health | Very high | NONE | |
| Odors | Very high | NONE | |
| Accidents | Very high | NONE | |
| Sea and ocean pollution | Very high | NONE | VERY SIGNIFICANT |
| River and lake pollution | Very high | NONE | VERY SIGNIFICANT |
| Water table pollution | Very high | NONE | VERY SIGNIFICANT |
| Nature and landscape | Very high | NONE | VERY SIGNIFICANT |
| Economical yield | Negligible | EXTREMELY SIGNIFICANT BY COMPARISON | VERY SIGNIFICANT |
| Investments | Extremely high | VERY LOW BY COMPARISON | |
| Secondary waste, slurry, and ashes | High and hazardous | NONE | |
| Power consumption | Very high | VEY LOW | |
| Reuse-recycling | Low and not cost effective | SAME CONDITIONS | |

Moreover, pig manure constitutes one of the most significant environmental problems in the farming sector, affecting climate change, water quality, and air quality. Since the 1990s, the sector has grown almost continuously, and the current census figures are close to twice those of 1990.

The intensive pig farming industry is generating an unprecedented environmental disaster in Spain and in other European countries. Today, it generates 15 times more excrements than meat, an amount enough to fill twenty-four thousand Olympic-sized swimming pools a year. This manure (a mixture of liquid and solid excrements) is responsible for aquifer and river pollution by nitrates.

Manure is usually deposited in thousands of open-air pools next to livestock farms in order to use them to fertilize fields. The problem occurs when those lands receive more manure than what they can absorb, causing overfertilization problems. Excess nitrate components leak into the earth, polluting aquifers that supply water to the population.

In addition to leakages due to excessive fertilization, manure pools also become destroyed and spill over in adverse climatic conditions. Intentional dumping into rivers and sewer system is also a common occurrence.

Moreover, according to a report issued by the World Health Organization, WHO, concerning the impact of the environment on health, 23% of deaths, which represent 12.6 million deaths per year, are related to the environment, 8.2 million of which are due to non-communicable diseases.

Poorer countries and people between 50 and 75 years of age are most affected by environmental problem, and children below 5 years of age suffer the worst consequences.

Therefore, an improvement in environmental care would have a direct and very significant repercussion on the health of the population.

Based on the foregoing, a new philosophy in waste treatment, storage, and management systems is required to stop the environmental deterioration of the planet.

### Description of the Invention

The invention solves the aforementioned problems by providing a technology capable of managing disposed waste without generating any type of environmental deterioration.

More specifically, one aspect of the invention relates to an integral waste treatment, storage, and management method in which physical, chemical, and biological treatments are combined, and it comprises the following operating phases:
crushing the waste and chemically treating the crushed waste with a chemical substance with bactericidal and/or biocidal properties,
compacting the chemically treated waste to form a compact block of waste,
enclosing the compact block of waste inside a flexible bag,
applying a vacuum inside the flexible bag, such that the block of waste is subjected to the vacuum to permanently prevent bacterial growth,
introducing the flexible bag with the compact block of waste therein into a rigid container,
hermetically closing the container, and
decontaminating the inside of the container by applying an inertization substance on the outside of the flexible bag and on the inner surface of the container, which substance eliminates any contamination that may have been produced on the outside of the flexible bag and the container while handling same before being introduced into the container,
such that the definitive storage of the waste inside the container is maintained in the absence of biological or chemical activity.

In the present description, the term inertized must be understood to mean any process which destroys the biological, physical, or chemical activity in the matter subjected to that process.

For applying a vacuum inside the flexible bag, the flexible bag has a valve suitable for creating and maintaining the vacuum therein.

In a preferred embodiment, the rigid container incorporates at least one valve in at least one of its panels for applying, from the outside, an inertization substance into the container on the outside of the flexible bag and the inner surface of the container inside the hermetically closed container. In other embodiments, depending on the size of the container and the substance to be used, several valves can be provided, and they can be arranged in several panels of the container so as to ensure that the inertization substance reaches the entire outer surface of the flexible bag and the inner surface of the container.

For that purpose, separating elements have been provided inside the container to form a space between the flexible bag and inner surface of the container so as to facilitate the circulation of the inertization substance throughout the inside of the container.

This inertization substance has bactericidal and/or biocidal properties and may consist of a gas, a liquid, or any other means suitable to that end. For example, the inertization substance may consist of ozone, caustic soda, or calcium oxide.

The rigid container is made of a non-biodegradable polymer material and the hermetic sealing thereof is performed by means of a suitable adhesive or by means of heat sealing. Alternatively, in other preferred embodiments of the invention, the hermetic closure of the container can be performed by means of ultrasounds or any other method suitable for this purpose.

The rigid container has a quadrangular prism shape, preferably a cubic shape, and has reinforcement ribs to prevent the buckling thereof, with these reinforcement ribs having been envisaged to also form the aforementioned separating elements. The block of waste is compacted to give it a quadrangular prism shape and sized to fit into the container and such that the separating elements keep the compact block of waste (housed in the flexible bag) separated from the inner walls of the container.

Preferably, the method of the invention uses non-recyclable waste: plastic waste, organic waste, and unsorted waste (waste not belonging to any predefined class) that are previously separated in a waste sorting phase and originate from land or marine waste. Furthermore, organic waste is previously dried before the crushing and compacting phase.

In a preferred embodiment of the invention, the rigid container has pointed elements at least on the inner face of several of its panels, the application of an inertization substance on the flexible bag inside the hermetically closed rigid container, alternatively or complementarily to the use of the valve of the container, is carried out by means of the following operating phases:
prior to hermetically closing the container, introducing a cushion or closed bag containing an inertization substance, which can be a gas, or a liquid, or other suitable means, into the container, and such that the bag is arranged between the pointed elements and the compact block of waste,
hermetically closing the container such that the compact block and the bag are housed therein, and
moving the container such that the bag comes into contact with the pointed elements and the breakage thereof is caused in order to release and apply the inertization substance on the flexible bag and the inner surface of the container.

The movement of the container is performed with suitable machinery and may consist of shaking it, causing it to vibrate, or tipping it over onto one of its faces to a sufficient degree so as to cause the breakage of the bag containing the inertization substance; for example, the movement can be such that the weight of the compact block of waste presses against the bag containing the inertization substance to assure the tearing thereof.

Before crushing the waste, it is separated into recyclable and non-recyclable waste, and non-recyclable waste is in turn separated into organic and inorganic waste. Organic waste is subjected to the following treatment: (i) applying ultraviolet radiation, (ii) drying in a heat tunnel, (iii) applying a chemical substance with bactericidal and/or biocidal properties, (iv) optionally crushing and compacting the waste, (v) introducing same in a flexible bag, (vi) applying a vacuum inside the flexible bag, and (vii) lastly, introducing the flexible bag into the rigid container and hermetically closing same.

One skilled in the art will understand that, in other preferred embodiments, the container may have any other shape suited to each application, such as a cylindrical shape or any polyhedral shape.

Finally, the containers are labeled by means of an identification code for the traceability of each container during its subsequent storage or use as a construction module.

In other embodiment variants of the invention, instead of enclosing a single compact block of waste inside a container, several smaller size blocks can be enclosed, and this, depending on each application, may have the advantage of facilitating the handling of smaller blocks, for example, manually if machinery for moving heavier blocks is not available.

Another aspect of the invention relates to a waste storage container suitable for carrying out the method described above. The container comprises rigid panels which are made of a non-biodegradable polymer material and can be coupled to one another to form a prismatic body, preferably a cubic body.

The panels have a plurality of separating elements on their inner face, and it optionally has at least one valve in at least one of the panels suitable for applying an inertization substance inside the container once it has been hermetically closed.

The panels are flat and have a perimetral edge, such that edge guards of the container can orthogonally couple and attach two panels along the edge thereof in order to assemble the container, such that once the container is assembled, the edge guards form the arrises of the prismatic body, and wherein the edge guards can be coupled to one another in the corners of the container.

Once it houses a block of waste therein and is hermetically closed, the container forms a construction module containing a biological, chemically, and physically inertized, compacted block of waste, such that the definitive storage of the waste inside the container is maintained permanently over time in inert conditions, i.e., in the absence of biological, chemical, or physical activity.

The compacted block of waste is spaced from the inner surface of the bases and side walls by means of the separating elements, so there is defined between both elements, the block of waste and the container, a space or chamber that facilitates the application of an inertization substance on the flexible bag and the inner surface of the container.

In a preferred embodiment of the container, all the panels forming the container are the same so as to simplify their manufacture.

Another aspect of the invention relates to a waste treatment and storage facility for carrying out the method of treatment described above and using the aforementioned container.

The facility comprises:
- means for selectively separating plastic waste, organic waste, and unsorted waste, and means for drying organic waste,
- means for biological sterilization of by means of ultraviolet radiation, and means for chemical sterilization by means of spraying or misting,
- crushing machinery and compacting machinery, provided for crushing and compacting previously sterilized waste so as to form a compact block of waste,
- means for introducing the compact block of waste into a flexible bag,
- means for applying a vacuum inside the flexible bag,
- means for introducing the compact block of waste into a rigid container like the one described above,
- means for hermetically closing the container, and
- means for applying an inertization substance inside the hermetically closed container, and
- preferably, means for moving, rotating, or tipping over the container once it is hermetically closed.

As can be seen, one of the main advantages of the method, container, and facility according to the invention is that the treatment and storage process is performed without any type of emission into the environment such as: gases, liquids, odors, or hazardous particles, and without waste accumulations in landfills.

Furthermore, the technology proposed in the invention constitutes a radical change in the conventional manner of dealing with waste treatment, since waste inertization and reuse is proposed in comparison with the conventional concept of incineration or accumulation in landfills, causing land, water, and air pollution.

The invention also constitutes a quick and efficient emergency solution in the event of temporary problems such as accidental waste spillage or seasonal increases in population, and accordingly in waste generation (a common problem in coastal areas during holiday periods). The intrinsic character of the technology of the invention conceived from the start of its design and based on process mobility and scalability allows it to be adapted to the suitable amount of waste generated in each location and time period, since it has been envisaged that all the machinery and equipment of the treatment facility are readily transportable to any location where needed in a matter of hours, which allows providing an immediate solution in the event of accidents and/or catastrophes.

For example, the technology of the invention could provide a quick, effective, and efficient solution to landfill spillage like the one that occurred in Zaldibar (the Basque Country), tire landfill fires like the one in Seseña (Castilla La Mancha), all times of contaminant waste accumulation in Mar Menor (Murcia), accumulated plastic islands with a surface area exceeding 1.7 million square km (the Pacific Ocean), etc.

### Brief Description of the Figures

Figure 1 schematically shows several of the processes making up the technology object of this invention. Specifically, Figure 1A offers an overview of the processes of the invention; Figure 1B shows a sequence of operating phases of the organic waste, plastic waste, and unsorted waste treatment process; and Figure 1C shows an operating sequence of the processes of the storage phase.
Figure 2 shows a perspective view of the waste container object of the invention.
Figure 3 shows an exploded view of the waste container object of the invention.
Figure 4 shows respective perspective views of the container closing process according to the invention for enclosing a compacted block of waste inside the container of the preceding figures using, in Figure 4A, a valve (without using a bag containing the inertization substance) and using, in Figure 4B, a bag containing the inertization substance. Figure 4A shows the content of the bag (12) by means of a partial section thereof.
Figure 5 shows an elevational section view of the container of Figure 4B once it is closed, prior to the breakage of the bag containing the inertization substance.
Figure 6 shows a depiction similar to that of Figure 5 after the breakage of the bag containing the inertization substance.
Figure 7 schematically shows the treatment facility or plant for putting the processes of the invention into practice.

### Description of a Preferred Embodiment of the Invention

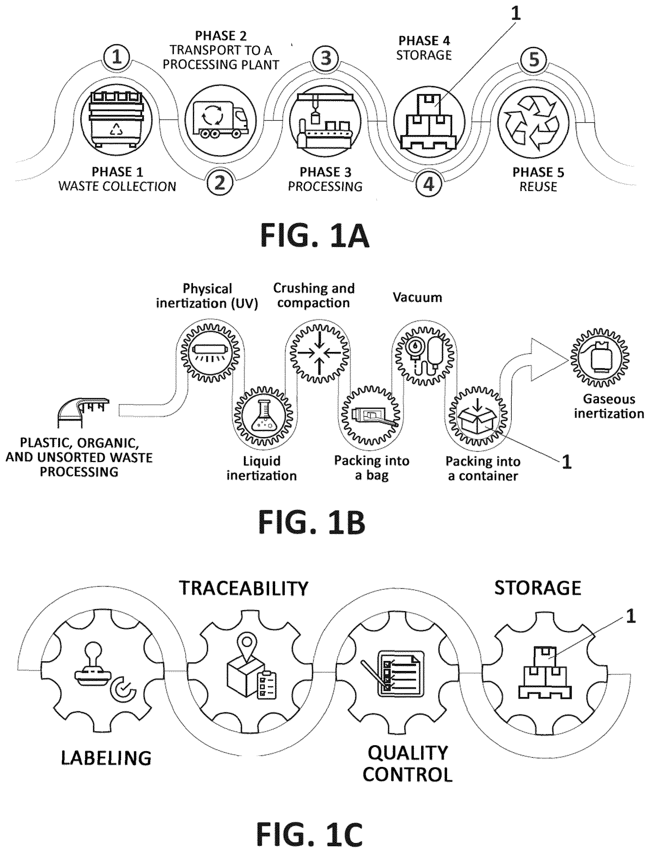

**Figure 1A** schematically shows the general sequence of the operating phases making up the method of the invention, which is made up of:
Phase 1. Waste collection. In this first phase, waste is collected from all the ecosystems, i.e., be it aquatic or land waste.
Phase 2. Transport to a processing plant. This phase is formed by the processes required for transporting all the waste collected in the preceding phase to its destination in the so-called processing plant reception area.
Phase 3. Processing according to the methodology and facility of the invention. This phase will include all the biological, physical, and chemical methods encompassing the tasks of selective waste separation, reuse, sterilization (biological inertization, liquid inertization), crushing, drying, compaction, isolation in a bag, vacuum, and storage in a container according to the invention.
Phase 4. Storage in the container (1) of the invention. This phase comprises the processes of verifying quality, labeling, traceability, and storage in an Eco Park, for example.
Phase 5. Reuse. This phase encompasses all the actions intended for the reuse, marketing, and/or effective and efficient destruction of each container and/or residual elements separated during the selective separation process carried out in the third phase.

The containers will be ready to be reused for a wide range of applications, whether for containment dam construction, structure construction, slope cover, breakwaters, filler, ballast for cranes or other machinery, ballast for ships, building, construction of architectural barriers, river channeling, perimetral fence, mine filler, etc.

The actual configuration of the containers, as well as the materials used in the manufacture thereof, the processes to which they have been subjected, and the different inertization processes carried out on their content, ensure that the containers are durable and stable over time, do not experience degradation, and are incapable of reacting and/or interacting with the media or any type of environmental pollution or impact.

Therefore, the initial starting point is waste collection from two completely different environments, i.e., the aquatic environment and the land environment, and a long list of categories of waste can be found in said environments, where it is even found that much of said waste may belong to several of these categories at the same time based on the selection criterion: biowaste, packaging, tires, slurry, oils, batteries, cells, mining waste, sanitary waste, wood, metals, plastics, home appliances, textile waste, glass, organic waste, and an endless number of categories that increases as human and material development progresses and based on the chosen sorting criteria.

In addition to the intrinsic danger of this waste, there is a need to contemplate that its collection implies the entraining of particles, bacteria, virus, and other microorganisms that are harmful to health, and so its processing and subsequent storage must undoubtedly involve an array of sterilization processes (referred to as inertization processes) that will be described in detail below. It is considered that the important task of sterilization which is required must be emphasized; several rather recent examples of the danger that the inexistence of sterilization or an inefficient sterilization process entails can be cited without having to go too far back in time.

During the global epidemic caused by coronavirus referred to as COVID-19, the existence of a high population rate of this virus in waste waters originating from human use, organic waste, and all types of surfaces and elements became apparent. There were even cases in which strains of the virus survived weeks-long sea voyages inside refrigerated containers. Without proper sterilization, not only local people but the entire world will be exposed to continuous and very serious epidemics.

Therefore, the technology developed in this invention covers all the aforementioned waste, with the exception of radioactive waste or waste whose physical/chemical composition may give rise to undesired physical/chemical reactions leading to the rupture of the container, and accordingly waste processing failure, i.e., it is estimated that the process of the invention is capable of treating ninety-nine point seven percent (99.7%) of the world's waste produced by humans, animals, and/or in agriculture.

Therefore, during the initial processing phase, the waste is selectively separated into eight categories: organic waste, glass waste, celluloid waste, plastic waste, metal waste, unsorted waste (waste not corresponding to any established category), electronic waste, rubber waste, gum waste, and organic waste. All the elements selectively sorted in the preceding categories of waste (with the exception of plastic waste, organic waste, and unsorted waste) are transported to the corresponding recycling and reuse plants, either in the compacted or non-compacted form depending on the requirements and availability of logistics means.

Once selectively separated, the plastic waste, organic waste, and unsorted waste are subjected to the processes that are listed above and shown in **Figure 1B****.** Specifically, in view of the preferred embodiment of **Figure 1B****,** it can be seen that the integral waste treatment, storage, and management method of the invention comprises at least the following operating phases:
- 1. Processing of the plastic waste, organic waste, and unsorted waste. First, the waste to be treated must be taken out of its initial storage container and deposited on a main conveyor belt. The waste on this automatic conveyor belt will go through different automatic and manual selection means, with an initial selective separation into six categories of waste being performed.
   The remaining waste which has not been segmented and removed from the initial conveyor belt by the automatic selection systems or manually by operators will correspond to two additional categories:
   - (i) Plastic waste and unsorted waste;
   - (ii) Organic waste.
   After the selection processes, sorted waste will be obtained, duly stored in containers ready to be moved to recycling and reuse plants suitable for each type of waste.
- 2. Physical and biological waste sterilization or inertization. Subsequently, there is arranged on the main conveyor belt (on which there remain the two categories of waste listed above) a biological inertization system for performing biological inertization by means of ultraviolet radiation, and the waste then goes through a liquid inertization system for performing liquid inertization by means of spraying and/or misting a liquid chemical agent that will be sprinkled on the waste before it is subjected to a last manual selection process in which the plastic waste will be separated before being stored in a container that will incorporate a grating in its lower part on a bilge towards which excess chemical agent will flow, and said bilge will allow storing said excess chemical agent for the reuse, treatment, and/or elimination thereof. The remaining waste (organic waste and unsorted waste) will reach the end of the conveyor belt, being stored in a container having similar characteristics as the plastic waste container.
- 3. Crushing and compacting the previously sterilized waste so as to form a compact block of waste. In this phase, the plastic waste and the organic waste will be ready to be subjected to a crushing process and a mechanical compaction process the purpose of which is to drastically reduce the volume they occupy, and accordingly their packing factor (compaction process). Once compacted, the plastic waste and the organic waste, as well as the unsorted waste, will be converted into a solid mass, preferably a biologically, physically, and chemically inertized, cubic mass.
- 4. Packing the compact block of waste in a flexible package or bag, such that it is isolated from the outside.
   Suitable for such functionality.
- 5. Creating a vacuum in the flexible bag through a valve in the bag provided for such functionality.
- 6. Packing the block of waste in a container (1) which is in turn made up of the phases of: introducing the compact block of waste (enclosed in the bag under vacuum) into a rigid container, hermetically closing the container, and applying an inertization substance, for example a gas, on the flexible bag and the entire inside of the container, such that the definitive storage of the waste inside the container is maintained in the absence of biological or chemical activity.

At this point, the waste will have reached a transformation state in which it forms a cubic solid mass having an identified overall composition that is biologically, physically, and chemically inertized (by means of UV radiation, liquid agent, and gaseous agent), subjected to vacuum and isolated from the outside by means of a first barrier (flexible bag) and by means of the rigid container.

The container that is ultimately obtained is a stackable, sturdy, cubic structure having known and standardized dimensions and a heterogeneous and identified overall composition, said structure being isolated and internally inertized and lasting for centuries.

As can be seen, at no time does the waste reach a landfill or undergo incineration, rather all waste that can be reused is destined for such purpose and waste that cannot be reused is processed and gathered in the containers according to the invention, and this causes the invention to have a greater ecological value and to be sustainable and efficient from an environmental viewpoint given that no gases, vapors, odors, or particles originating from waste incineration are emitted and no uncontrolled accumulations in the form of landfills with a potential risk of biological contamination, leachate production, and/or other environmental hazards, are produced.

The level of recycling and reuse is maximum and optimum, the final storage volume of waste that is treated (and accordingly harmless for the environment and can be used again) will be very considerably reduced, and the overall processing costs per kg of waste are considerably much lower.

**Figure 1C** shows the processes of the storage phase which is made up of the phases of: labeling the container, traceability, quality control, and sending for reuse as a construction or storage module in an ECO PARK (ecological park for waste).

The first process (labeling process) may consist, for example, of assigning a unique alphanumerical code to each container, recorded on each of the four side faces of the container. Associated with this alphanumerical code, all the parameters associated with the container (traceability process) such as the manufacturing date, collection date, nature of its content, the processes used, etc., will be recorded by computers.

The container is then subjected to a quality inspection process based on manual and/or automatic physical and optical means in order to verify compliance with the required methods and to verify the quality of the defined standards. Lastly, a process of sending the container to an ECO PARK for reuse (when the content is reusable, for example, for composting or construction, among other applications) or storage will be performed.

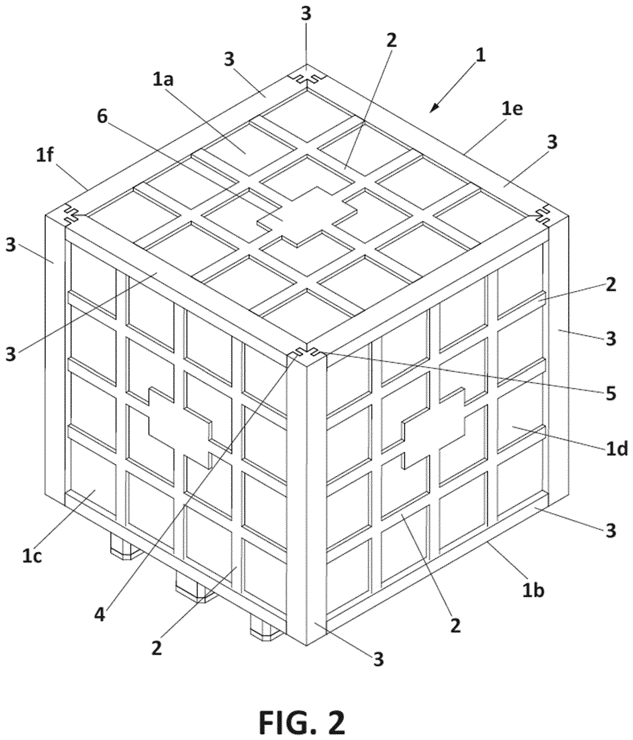

A preferred embodiment of the waste storage container (1) for carrying out the processing method described above has been depicted in **Figures 2** to **6****.**

Specifically, in view of **Figure 2****,** it can be seen that the container (1) comprises several panels, particularly an upper base and a lower base (1a, 1b), and four side walls (1c, 1d, 1e, 1f) that can be coupled to one another to form a prismatic body, preferably a cubic body, like the one depicted in **Figure 2****.**

The upper and lower bases and side walls (1a - 1f) are flat, have a square shape, and reinforcement ribs (2) on both the inner face and outer face thereof in order to provide the necessary structural rigidity to the faces of the container (1). As can be seen in the figure, the ribs (2) are arranged forming a rib framework or network, with a central plate (6) on each of the faces of the container (1) to inscribe an identification code or any other information deemed necessary.

The reinforcement ribs (2) are furthermore sized to form separating elements inside the container (1) as will be described below.

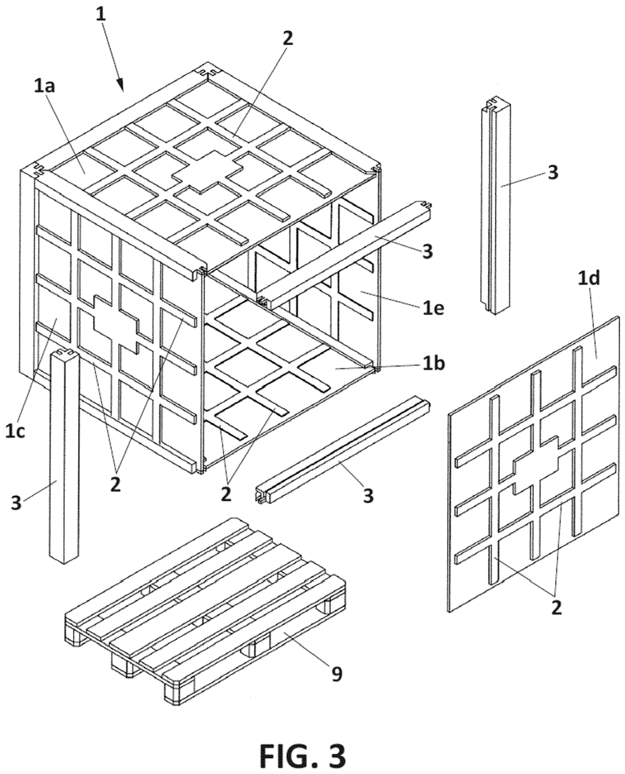

The container (1) furthermore incorporates edge guards (3) intended for forming the twelve arrises of the cube-shaped container, for which the edge guards (3) have channels (4, 5) orthogonal to one another for the insertion, by way of tongue-and-groove, of the edges of two walls, and an adjacent wall and base, respectively, and for orthogonally attaching both as depicted in **Figure 3****.**

Furthermore, the edge guards (3) are configured for being attached to one another in the corners of the container (1), also by way of tongue-and-groove, as can better be seen in **Figure 2****.**

As can be seen, the manufacture, storage, and transport of the components forming the container (1) are extremely efficient, since the panels can be stacked, taking up very little space.

Preferably, the bases and side faces (1a - 1f) have the same shape and size, so container manufacture and assembly are greatly simplified. The edge guards (3) also have the same shape and size so as to simplify container manufacture and assembly.

The edge guards (3) and panels, i.e., the bases, side faces (1a- 1 f), are manufactured with the same material, preferably with a non-biodegradable polymer material.

It is envisaged that the components of the container can be manufactured by 3D printing, or in other preferred embodiments, it is envisaged that several parts of the container can be manufactured as a single part by 3D printing.

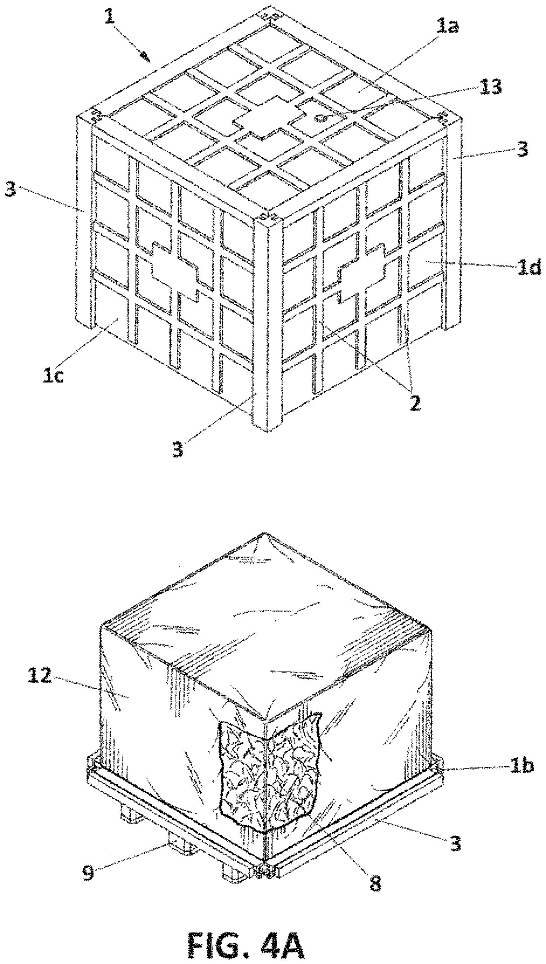

The use of the container (1) as part of the treatment method described above has been depicted in **Figures 4A****,** **4B****,** **5****,** and **6****.**

Specifically, in view of **Figure 4A****,** a compact block (8) of waste which has been biologically, chemically, and physically inertized according to the process described above, and enclosed under vacuum inside a flexible package or bag (12), is placed on the lower base (1b) of the container (1) which in turn rests on a standard pallet (9).

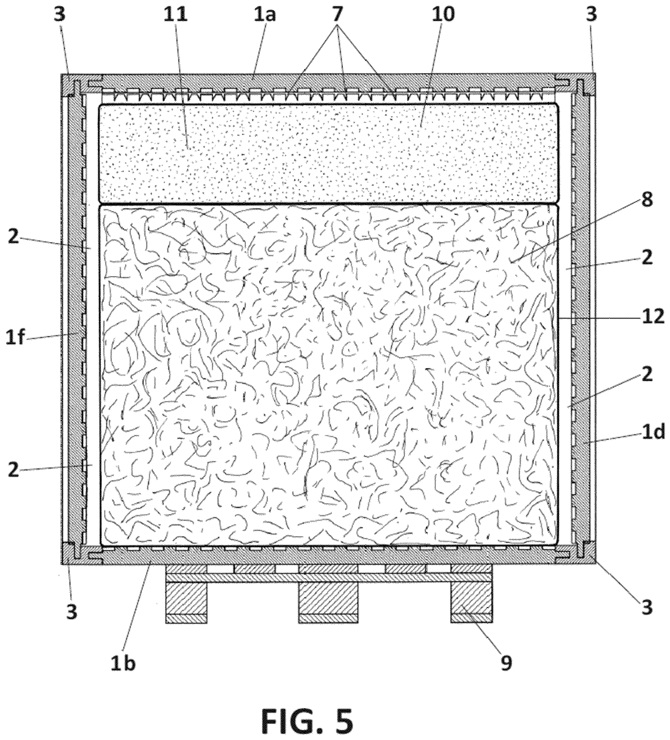

The rest of the pre-assembled container, i.e., the upper base and side faces (upper part of **Figure 4A**), is lowered for coupling with the lower base (1b), leaving the compact block (8) enclosed inside the container (1) as shown in **Figure 5****,** with the hermetic sealing of the container being performed thereafter, and such that the compact block is retained and separated from the inner faces of the container (1) by means of the reinforcement ribs (2).

The reinforcement ribs (2) further acting as separators form a space between the bag (12) and all the inner faces of the container (1) so that the inertization substance can reach the entire outer surface of the bag (12) and inner surface of the container (1).

In the case of **Figure 4A****,** one of the panels of the container has a valve (13) for injecting an inertization substance into the container (1) from the outside so as to decontaminate the outer surface of the bag (12) and the inner surface of the container, and to thereby ensure that there will be no bacterial activity whatsoever inside the container. After this operation of injecting the substance into the container, the valve is closed, ensuring the hermetic nature of the container.

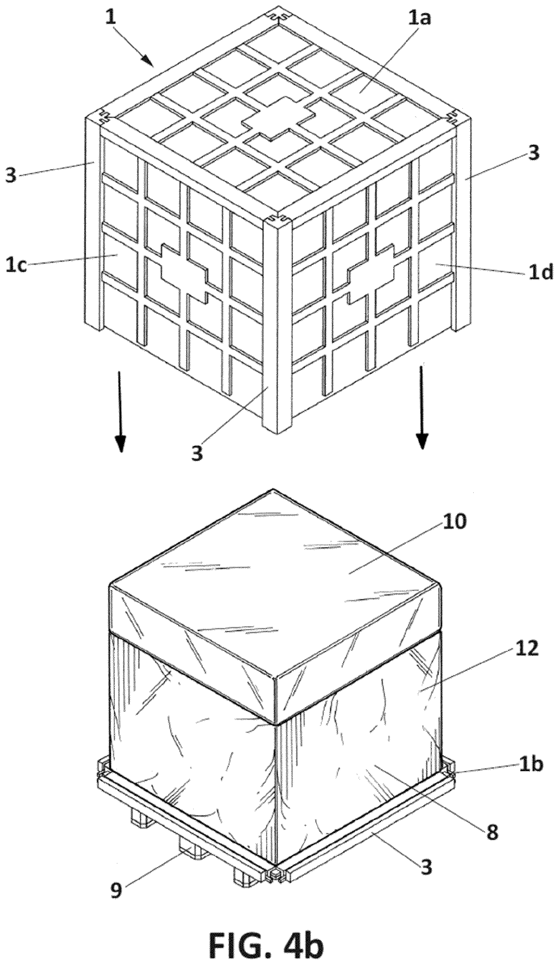

Alternatively or complementarily to the use of the valve, according to the embodiment variant of **Figure 4b****,** one of the panels of the container, in this case the upper panel (1a), has pointed elements (7) on its inner face, and a closed bag (10) containing an inertization substance (11) is placed on the compact block (8) of waste, such that the bag (10) is arranged between the pointed elements (7) and the compact block (8) of waste, as seen in **Figure 5****.**

Preferably, the pointed elements (7) are not part of the panel of the container, but rather consist of separate components that are fixed by any conventional means to the inner face of one of the panels.

The container (1) can be sealed by means of any known technique, preferably using a suitable adhesive applied in the channels (4, 5) of the edge guards (3).

The compact block (8) is formed and sized so as to be able to be received inside the container in the space made available by the reinforcement ribs (2), leaving a space inside the container for the bag (10) containing the inertization substance.

In other preferred embodiments, the compact block (8) is sized such that the bag (10) with inertization gas can be placed on one side of the compact block (8) instead of on its upper base.

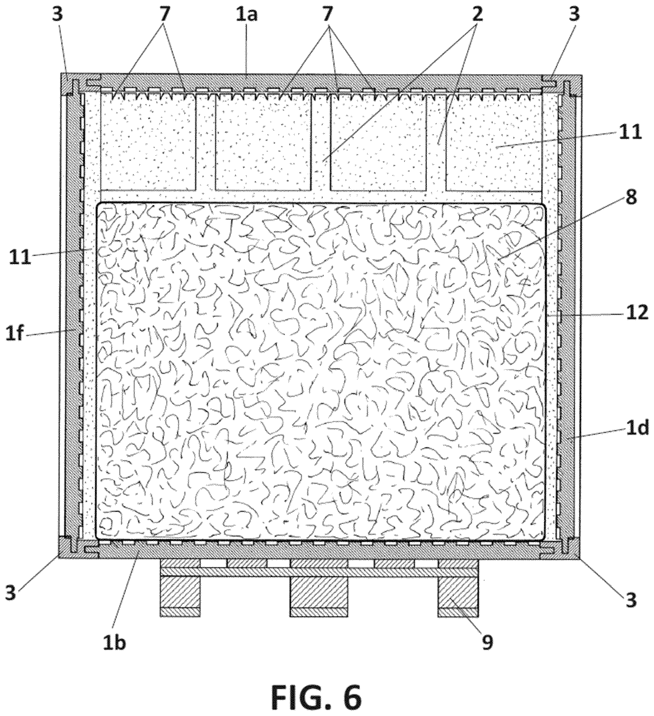

In the situation of the container of **Figure 5****,** in order to decontaminate the bag (12) inside the container (1) and the inner surfaces of the container (1), there is a need to break the bag (10) by means of the pointed elements (7) by moving the container (1), whether by shaking, rotating, or tipping over the container so that the bag (10) contacts the pointed elements (7) and starts to tear, releasing the inertization substance (11) which is dispersed throughout the inside of the container as illustrated in **Figure 6****.**

In this embodiment, proper care must be taken to keep the container (10) upright, as shown in **Figure 6****,** so that the pointed elements (7) do not accidentally break the bag (12) and cause the vacuum to disappear.

In other preferred embodiments, the valve system of **Figure 4A** and the cushion or closed bag system of **Figure 4B** can be used in order to utilize, for example, two different types of inertization substances that further assure the decontamination of the inside of the container.

Waste storage in completely inert conditions is thereby assured by the maintenance of the (previously physically, chemically, and biologically inertized) waste under vacuum conditions, and furthermore by the bacteriological decontamination of the outside of the flexible bag and the inner surface of the container, so the waste can be stored indefinitely in inert conditions, i.e., in the absence of biological or chemical or physical activity.

In other preferred embodiments, the container (1) has anchoring means (not depicted) for anchoring several containers to one another to form larger geometrical structures, reducing the final storage volume, increasing the total mass of the container assembly, and allowing the centers of gravity and the centers of masses of the assembly to be changed according to storage and/or meteorological requirements, or containment means to be dealt with in a quick, efficient, and intuitive manner in the event of adverse meteorological conditions, for example, rising rivers or dams.

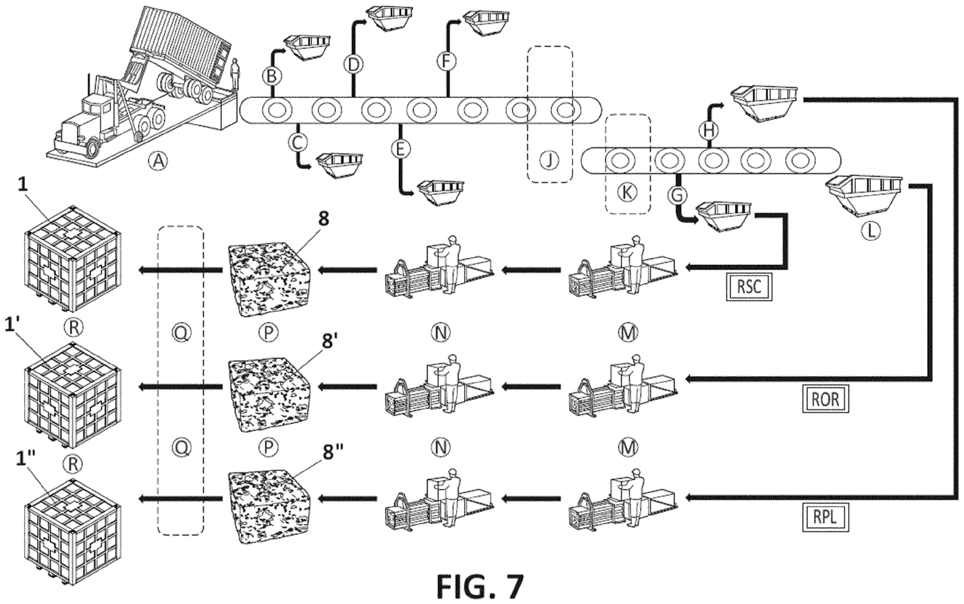

An embodiment of the waste treatment and storage facility or plant for carrying out the treatment method described above has been depicted in **Figure 7****.**

The processing plant is the vital center of the entire process because this is the point of arrival of waste which will initially be stored, treated, sorted, distributed to reuse/recycling plants, and ultimately subjected to an inertization process and stored in a completely isolated manner from waste the composition of which does not allow it to be reused.

The facility is designed as a temporary plant that can be removed, replicated, and moved, i.e., it has a general size and arrangement allowing it to be quickly installed in all types of locations, to be quickly taken down, without leaving any trace of its presence, and to be transported from one place to another where its presence is required.

The facility comprises:
means for selectively separating plastic waste, organic waste, and unsorted waste, and means for drying organic waste,
means for biological sterilization of by means of ultraviolet radiation, and means for chemical sterilization by means of spraying or misting,
crushing machinery and compacting machinery, provided for crushing and compacting previously sterilized waste so as to form a compact block of waste,
means for introducing the compact block of waste into a flexible bag,
means for applying a vacuum inside the flexible bag,
means for introducing the compact block of waste into a rigid container, preferably the container (1) described above,
means for hermetically closing the container, and
means for distributing an inertization substance inside the hermetically closed rigid container.

More specifically and in view of **Figure 7****,** it can be seen that the facility has the following stations.
Station (A). Waste reception, for example, in a truck.
Station (B). Metal waste separation.
Station (C). Glass waste separation.
Station (D). Celluloid waste separation.
Station (E). Electronic waste separation.
Station (F). Gum and rubber waste separation.
Station (G). Unsorted waste separation.
Station (H). Plastic waste separation.
Station (J). Physical inertization (ultraviolet light).
Station (K). Liquid inertization (misting).
Station (L). Organic waste separation and drying.
Station (M). Crushing.
Station (N). Compaction.
Station (P). Packing in a flexible package (bag).
Station (Q). Vacuum.
Station (R). Packing in a container (1, 1', 1").
(RSC). Secondary conveyor belt for unsorted waste.
(ROR). Secondary conveyor belt for organic waste.
(RPL). Secondary conveyor belt for plastic waste.

## Claims

1. A method for integral waste treatment, storage, and management which comprises:
crushing the waste and chemically treating the crushed waste with a chemical substance with bactericidal and/or biocidal properties,
compacting the chemically treated waste to form a compact block of waste,
enclosing the compact block of waste inside a flexible bag and applying a vacuum inside the flexible bag,
introducing the flexible bag with the compact block of waste therein into a rigid container,
hermetically closing the container, and
applying an inertization substance inside the hermetically closed container to decontaminate the outside of the flexible bag and inner surface of the container,
such that the definitive storage of the waste inside the container is maintained in the absence of biological or chemical activity.

2. The method according to claim 1, wherein the rigid container has at least one valve suitable for applying an inertization substance therein.

3. The method according to claim 1 or 2, wherein the flexible bag has a valve suitable for creating and maintaining the vacuum therein.

4. The method according to any of the preceding claims, wherein the rigid container is made of a non-biodegradable polymer material, and in that the container is hermetically closed by means of an adhesive or heat sealing.

5. The method according to any of the preceding claims, wherein the container has separating elements on its inner faces, and wherein the block of waste is compacted and sized to fit into the container and such that the separating elements keep the compact block of waste, housed in the flexible bag, separated from the inner walls of the container, forming a space between both so that the inertization substance can be applied around the flexible bag and the inner walls of the container.

6. The method according to claim 5, wherein the rigid container has a quadrangular prism shape and wherein the block of waste is compacted to give it a quadrangular prism shape and sized to fit into the container.

7. The method according to any of the preceding claims, wherein the rigid container has pointed elements on at least one of its faces, and wherein the application of an inertization substance on the flexible bag inside the hermetically closed rigid container, alternatively or complementarily to the use of the valve of the container, is carried out by means of the following operating phases:
prior to hermetically closing the container, introducing a closed bag containing an inertization substance into the container, and such that the bag is arranged between the pointed elements and the compact block of waste,
hermetically closing the container such that the compact block and the bag are housed therein, and
moving the container such that the bag comes into contact with the pointed elements and the breakage thereof is caused in order to release the inertization substance on the flexible bag and the inner surfaces of the container.

8. The method according to the preceding claims, wherein prior to crushing the waste, the waste is separated into organic waste and non-organic waste, and in that the organic waste is subjected to the following treatment: (i) applying ultraviolet radiation, (ii) drying in a heat tunnel, (iii) applying a chemical substance with bactericidal and/or biocidal properties.

9. A container for waste storage, comprising panels that can be coupled to one another to form a prismatic body, wherein the panels are rigid and made of a non-biodegradable polymer material, and wherein two or more panels have a plurality of separating elements on their inner face, and it optionally has a valve in one of the panels suitable for applying an inertization substance inside the container once it has been hermetically closed.

10. The container according to claim 9, wherein the panels are flat and have a perimetral edge, and in that the container further comprises edge guards configured for orthogonally coupling and attaching two panels along the edge thereof in order to assemble the container, such that once the container is assembled, the edge guards form the arrises of the prismatic body, and wherein the edge guards can be coupled to one another in the corners of the container.

11. The container according to any of claims 9 or 10, furthermore having pointed elements on the inner face of at least one of the panels.

12. The container according to any of claims 9 to 11, which is hermetically closed and contains therein a compacted block of waste enclosed inside a flexible bag under vacuum conditions, and wherein the outer surface of the flexible bag has been treated with an inertization substance, and wherein the compacted block of waste is spaced from the inner face of the panels by means of the separating elements.

13. The container according to any of claims 9 to 11, wherein the panels have reinforcement ribs on their inner face or on the inner face and the outer face, and in that the reinforcement ribs are sized to form the separating elements.

14. A waste treatment and storage facility for carrying out the method of claims 1 to 8, comprising:
means for selectively separating plastic waste, organic waste, and unsorted waste, and means for drying organic waste,
means for biological sterilization of by means of ultraviolet radiation and means for chemical sterilization by means of spraying or misting,
crushing machinery and compacting machinery, provided for crushing and compacting previously sterilized waste so as to form a compact block of waste,
means for introducing the compact block of waste into a flexible bag,
means for applying a vacuum inside the flexible bag,
means for introducing the compact block of waste into a rigid container, preferably the container defined in claims 9 to 13,
means for hermetically closing the container, and
means for applying an inertization substance inside the hermetically closed container.

15. The facility according to claim 14, further having means for moving, rotating, or tipping over the container once it is hermetically closed.
